# EUROPEAN PATENT SPECIFICATION

(11) **EP 3 760 707 B1**
(45) Date of publication and mention of the grant of the patent: **19.11.2025**
(21) Application number: 20186304.0
(22) Date of filing: 08.11.2012
(51) Int. Cl.: C12N 5/00, A61K 35/33, A61P 19/02, A61K 45/00, A61K 45/06

(54) **FIBROBLASTS FOR USE IN THE TREATMENT OF DEGENERATIVE DISC DISEASE**
FIBROBLASTEN ZUR VERWENDUNG IN DER BEHANDLUNG VON DEGENERATIVEN BANDSCHEIBENERKRANKUNGEN
FIBROBLASTES ET LEUR UTILISATION DANS LE TRAITEMENT D'UNE MALADIE DISCALE DÉGÉNÉRATIVE

(30) Priority: 09.11.2011 US 201161557479 P; 21.08.2012 US 201261691391 P
(43) Date of publication of application: 06.01.2021
(62) Divisional of application: 17192969.8
(73) Proprietor: FibroBiologics, Inc., Houston, TX 77598 (US)
(72) Inventor: O'HEERON, Pete, Houston, TX Texas 77059 (US); AN, Howard, Glenview, IL Illinois 60026 (US)
(74) Representative: J A Kemp LLP

(56) References cited:
- WO-A1-2009/048675
- WO-A2-2014/204806
- US-A- 5 206 023
- US-A1- 2009 068 270
- US-B2- 7 850 983
- US-B2- 8 043 614
- MARIJNISSEN A C A ET AL: "Articular cartilage repair: Basic science and clinical progress. A review o f the current status and prospects", OSTEOARTHRITIS AND CARTILAGE, ELSEVIER, AMSTERDAM, NL, vol. 11, no. 4, 1 April 2003 (2003-04-01), pages 300 - 304, XP002419619, ISSN: 1063-4584, DOI: 10.1016/S1063-4584(02)00346-1
- FUSZ ET AL: "Characteristics of human chondrocytes, osteoblasts and fibroblasts seeded onto a type I/III collagen sponge under different culture conditions", ANNALS OF ANATOMY, JENA, DE, vol. 182, no. 4, 1 July 2000 (2000-07-01), pages 303 - 310, XP005033660, ISSN: 0940-9602
- MIZUNO SHUICHI ET AL: "Chondroinduction of human dermal fibroblasts by demineralized bone in three-dimensional culture", EXPERIMENTAL CELL RESEARCH, ELSEVIER, AMSTERDAM, NL, vol. 227, no. 1, 1 January 1996 (1996-01-01), pages 89 - 97, XP002234904, ISSN: 0014-4827, DOI: 10.1006/EXCR.1996.0253
- MENACHE D: "Prothrombin complex concentrates: clinical use", 19810101, vol. 370, 1 January 1981 (1981-01-01), pages 747 - 56, XP002015681
- S. CHANDRA ET AL: "Prothrombin Complex Concentrates for Clinical Use", VOX SANGUINIS, vol. 41, no. 5-6, 1 January 1981 (1981-01-01), CH, pages 257 - 273, XP055446030, ISSN: 0042-9007, DOI: 10.1159/000460659
- D-Y KIM ET AL: "G-I. Im, MD Repair of cartilage defect in the rabbit with cultured mesenchymal stem cells from bone marrow", J BONE JOINT SURG, vol. 83-B, no. 2, 1 January 2002 (2002-01-01) - 1 January 2001 (2001-01-01), pages 289 - 294, XP055183038, Retrieved from the Internet <URL:http://www.bjj.boneandjoint.org.uk/content/83-B/2/289.full.pdf> [retrieved on 20150415]
- SUDO KAZUHIRO ET AL: "Mesenchymal progenitors able to differentiate into osteogenic, chondrogenic, and/or adipogenic cells in vitro are present in most primary fibroblast-like cell populations", STEM CELLS (MIAMISBURG), vol. 25, no. 7, July 2007 (2007-07-01), pages 1610 - 1617, XP002738446, ISSN: 1066-5099
- S KADIYALA ET AL: "Original Contribution CULTURE EXPANDED CANINE MESENCHYMAL STEM CELLS POSSESS OSTEOCHONDROGENIC POTENTIAL IN VIVO AND IN VITRO", CELL TRANSPLANTATION COPYRIGHT, 1 January 1997 (1997-01-01), pages 125 - 134, XP055183039, Retrieved from the Internet <URL:http://www.sciencedirect.com/science/article/pii/S0963689796002795/pdf?md5=6cb04eeadbbb2cc085f426d6322c26f3&pid=1-s2.0-S0963689796002795-main.pdf> [retrieved on 20150415]
- SINGH M ET AL: "Chondrogenic differentiation of neonatal human dermal fibroblasts encapsulated in alginate beads with hydrostatic compression under hypoxic conditions in the presence of bone morphogenetic protein-2", JOURNAL OF BIOMEDICAL MATERIALS RESEARCH - PART A 20110901 JOHN WILEY AND SONS INC. USA, vol. 98 A, no. 3, 1 September 2011 (2011-09-01), pages 412 - 424, XP002738447, DOI: 10.1002/JBM.A.33129

## Description

### TECHNICAL FIELD

The field of the present invention concerns compositions for treatment of an individual in need of cartilage repair.

### BACKGROUND

Intervertebral discs, which may be referred to as intervertebral fibrocartilage, are positioned between adjacent vertebrae in the spine, and each disc forms a cartilaginous joint to permit slight movement of the vertebrae, acting as a ligament to hold the vertebrae together. Intervertebral discs comprise plates of fibrocartilage that correspond to the shape of the endplate surfaces of the vertebral bodies. The discs play a considerable role in weight bearing. Intervertebral discs comprise an outer annulus fibrosus, which surrounds the internal gelatinous nucleus pulposus. The annulus fibrosus insert into the smooth, rounded rims on the endplate surfaces of the vertebral bodies. The nucleus pulposus contact the hyaline cartilage plates, which are attached to the rough surfaces of the vertebral bodies.

Invertebral Disc Disease, which may be referred to as Intervertebral Disc Disorder and includes Degenerative Disc Disease (DDD), is a medical condition wherein there is dysfunction of the disc, including deterioration and/or herniation, for example. In DDD, there is gradual dehydration of the nucleus pulposus. Following this, the loads normally absorbed by the nucleus pulposus are instead transferred non-uniformly through the annulus fibrosus, which can undergo progressive, structural deterioration. Herniated discs (which may be referred to as slipped disc, ruptured disc, or a bulging disc) occur when the annulus fibrosus tears because of an injury or because of aging, upon which the nucleus pulposus can begin to extrude through the tear.

US 2009/068270 A1 discloses treatment of degenerated discs with autologous cells. Methods and compositions that are effective and have minimal invasiveness and/or preparation time for treating Invertebra1 Disc Disease are needed in the art.

### BRIEF SUMMARY OF THE DISCLOSURE

The present disclosure is directed to systems, methods, and compositions for treatment of an individual in need thereof, including treatment of an individual in need of cartilage repair. The present disclosure concerns methods and compositions for biological repair of any kind of cartilage, including intervertebral and joint cartilage, for example. In particular aspects, the present invention concerns the fields of cartilage repair, such as articular cartilage repair. Other aspects described herein include methods for growing, proliferating, and/or differentiating cells into chondrocyte-like cells under mechanical stress.

In certain aspects, the invention generates natural tissue in vivo from fibroblasts. Also described herein is a method for growing and differentiating human fibroblasts into chondrocyte-like cells, for example. The cells may be autologous or allogeneic or a mixture thereof, in certain aspects described herein.

In specific aspects, the disclosure employs differentiation of certain cells into chondrocyte-like cells. In specific aspects of the disclosure, human dermal fibroblasts (HDFs), for example, are differentiated into chondrocyte-like cells under particular conditions. Differentiation of cells into chondrocytes or chondrocyte-like cells may occur in any suitable manner, including *in vivo* following implantation.

Specific aspects described herein provide a method for in vivo regeneration of a joint, such as an intervertebral disc, elbow, knee, shoulder, hip, temporo-mandibular joint, and so forth.

In certain embodiments, the cartilage that is the focus of application of the invention is intervertebral disc cartilage. In particular aspects of the invention, cells utilized in the invention are subjected to *in vivo* mechanical strain for chondrogenic differentiation.

It is an exemplary object of the present disclosure to provide a method intended to repair a degenerated intervertebral disc, e.g. restore intervertebral disc anatomy and improve its functioning. Particular aspects described herein provide a method to repair damaged disc. In one aspect described herein, there is a method of repairing damaged cartilage in a joint (such as an intervertebral disc) of an individual, comprising delivering fibroblasts in accordance with the invention to the respective joint (such as intervertebral disc) of the individual. In specific aspects described herein, fibroblasts are delivered to the intervertebral disc in the absence of removing part or all of the degenerated disk.

Under mechanical stress, the provided cells will acquire the characteristics of nucleus cells in the central part and annulus cells in the periphery, for example. Exemplary fibroblast cells may be harvested from skin, such as by a biopsy, for example.

In certain aspects described herein, an individual is provided another therapy in addition to the methods described herein. For example, before, during, and/or after delivery of the fibroblast cells, the individual may receive one or more antibiotics. Exemplary post-operative therapies includes Non Steroidal Anti-Inflammatory Drugs (NSAIDs), simple pain killers (analgesics), and/or muscle relaxants as needed, and it may be followed by a functional rehabilitation post-operatively, such as after the first, second, third or more post-operative week, for example. In specific aspects, the individual may be provided one or more of an antibiotic, antifungal agent, or antiviral agent.

In certain aspects of the disclosure, the cells differentiate into chondrocyte cells or chondrocyte-like cells, such as wherein the chondrocyte cells or chondrocyte-like cells secrete a molecule selected from the group consisting of aggrecan, type II collagen, Sox-9 protein, cartilage link protein, perlecan, and combinations thereof. In particular cases, the cells are differentiated from fibroblast cells, and exemplary fibroblast cells include dermal fibroblasts, tendon fibroblasts, ligament fibroblasts, synovial fibroblasts, foreskin fibroblasts, or a mixture thereof.

In specific aspects described ehrein, there are no growth factors provided to the fibroblasts before, during, or after delivery in vivo to the individual in need thereof, including growth factors such as bone morphogenetic protein 2 (BMP-2), BMP-4, BMP-6, BMP-7, cartilage-derived morphogenetic protein (CDMP), transforming growth factor beta (TGF-β), insulin growth factor one (IGF-I), fibroblast growth factors (FGFs), basic fibroblast growth factor (bFGF), FGF-2, platelet-derived growth factor (PDGF), and a mixture thereof.

Further described herein is a kit comprising fibroblasts that are housed in one or more suitable containers. In specific aspects of the disclosure, the kit further comprises one or more reagents suitable for enhancing in vivo differentiation from fibroblasts to chondrocytes or chondrocyte-like cells. In some aspects of the disclosure, the kit includes one or more apparatuses for delivery of fibroblasts to an individual.

In some aspects of the disclosure, there are methods and compositions related to delivering fibroblasts to a site in vivo in an individual in need thereof. In specific aspects of the disclosure, the site is *in vivo* and in need of chondrocytes, including in need of cartilage. For example, a site in need of chondrocytes includes joints, for example cartilaginous joints (e.g., vertebrae). In some aspects disclosed herein for illustrative purposes, the fibroblasts are obtained from the individual in need of cartilage. In specific aspects of the disclosure, fibroblasts are delivered to at least one intervertebral disc in an individual. In some cases, the fibroblasts are manipulated following being obtained, whether or not they are obtained from the individual in need thereof or whether or not they are obtained from a third party or commercially, for example. The fibroblasts may be expanded in culture. In certain aspects of the disclosure, the fibroblasts are not provided growth factors, matrix molecules, mechanical strain, or a combination thereof, prior to or during or following implantation into a vertebrae.

In some aspects of the disclosure, there are both fibroblasts and chondrocytic cells in the disc. In some aspects of the disclosrue, not all fibroblasts that are delivered in vivo will differentiate to chondrocytes in the disc, yet the fibrous tissues that are produced in the disc are nevertheless useful in improving the disc height and biomechanical function.

Described herein is a method of differentiating human dermal fibroblasts into chondrocyte-like cells in vivo, comprising the step of delivering fibroblasts to a joint of an individual, wherein prior to delivering the fibroblasts are not subjected to growth factors, matrix molecules, mechanical strain, or a combination thereof. In specific cases, the individual has intevertebral disc disease. In some cases, the joint is an invertebral disc.

In some aspects of the disclosure, some of the undifferentiated fibroblasts and differentiated chondrocyte-like cells in the disc are further defined as cells that produce fibrous matrix molecules, cartilaginous matrix molecules, or both. In certain aspects, the chondrocyte-like cells are further defined as cells that produce matrix molecules, such as collagen I, collagen II, proteoglycan, or a combination thereof. In specific aspects of the disclosure, the collagen comprises type I and type II collagen. In some cases, one of the proteoglycans is aggrecans.

In particular cases, the fibroblasts are delivered between invertebral discs. In certain cases, the fibroblasts are delivered between or in nucleus pulposus and fissures in the inner annulus fibrosus. The fibroblasts may be delivered between invertebral discs, including nucleus pulposus and fissures in the inner annulus fibrosus, for example.

Some aspects of methods described herein for illustrative purposes include obtaining fibroblasts from the individual. The obtaining may encompass removal of fibroblasts from a body or may encompass retrieving already-obtained fibroblasts, such as from a third party, including commercially, or from storage, for example. In certain aspects of the disclosure, the fibroblasts are expanded, for example for at least one day. In some cases, the obtained fibroblasts are passaged, for example more than once. In particular aspects of the disclosure, the fibroblasts are both expanded and passaged.

In some aspects of the disclosure, there is a method of producing fibrous tissue and/or chondrocytic tissue in a joint of an individual, comprising the step of delivering fibroblasts to the joint, wherein the fibroblasts have not been exposed to growth factors, matrix molecules, mechanical strain, or a combination thereof, in vitro prior to or during or following delivery to the joint. In specific aspects of the disclosure, the fibrous and/or chondrocytic tissue comprise cells having particular biochemical markers, such as both type I and type II collagen and/or a number of proteoglycans found in cartilaginous and fibrous tissues, for example.

In certain aspects described herein, the presence of the fibroblasts and/or the death of fibroblasts before and/or after delivery to the joint of the individual triggers response from one or more cells. In specific cases, the presence of the fibroblasts and/or the death of fibroblasts triggers response from other cells in the joint, and the other cells may be of any kind, including the individual's endogenous cells, such as chondrocytes, fibroblasts, disc stem cells, etc. In particular aspects, the endogenous cell response includes stimulation of growth, for example as at least some fibroblasts die in the joint. Thus, in specific aspects the mere presence of the fibroblasts and/or release of intracellular factors upon death of cells may stimulate a cell growth response from existing cells in the disc. In particular cases, the cell growth response results in re-growth of the disc (or repair of the joint).

In particular aspects of the disclosure, as an indirect or direct result of delivery of the fibroblasts to the joint, scar tissue may form in the joint. In at least specific cases, such scar tissue formation is beneficial to the joint, for example when the joint is a disc, by providing stability, strength, cushion, seal of annular fissure(s) and so forth.

### SUMMARY OF THE INVENTION

The invention is defined in the independent claim. In particular, the invention provides fibroblasts for use in a method of treating an individual in need of cartilage repair, the method comprising differentiating human fibroblasts into chondrocyte-like cells in vivo by delivering fibroblasts to a joint of an individual, wherein prior to delivery, the fibroblasts are not subjected to growth factors, matrix molecules, mechanical strain, or a combination thereof. Further aspects of the invention and preferred embodiments are defined in the dependent claims. Any aspects, embodiments and examples of the present disclosure which do not fall under the scope of the appended claims do not form part of the invention and are merely provided for illustrative purposes.

### DETAILED DESCRIPTION OF THE INVENTION

As used herein the specification, "a" or "an" may mean one or more. As used herein in the claim(s), when used in conjunction with the word "comprising", the words "a" or "an" may mean one or more than one. As used herein "another" may mean at least a second or more.

Embodiments of the invention may "consist essentially of" or "consist of" one or more elements or steps of the invention, for example.

It is contemplated that any method or composition described herein can be implemented with respect to any other method or composition described herein.

The term "chondrocyte-like cells" as used herein refers to cells that are not primary chondrocytes but are derived from fibroblasts, for example. These chondrocyte-like cells have a phenotype of chondrocytes (cells of cartilage) including a shape of chondrocytes (polygonal and/or rhomboidal cells, for example) and/or are able to aggregate and produce cartilage matrix components, such as sulfated proteoglycan and type II collagen, for example. Thus, exemplary markers of chondrocyte-like cells include one or more of aggrecan, which is a chondroitin sulfate and keratan sulfate proteoglycan, type II collagen, Sox-9 protein, cartilage link protein, and perlecan, which is a heparan sulfate proteoglycan, for example.

The term "joint" as used herein refers to a region in the body wherein two bones of a skeleton join.

Although any tissues may be repaired at least in part by methods described herein, including any cartilage tissues, in a particular exemplary aspect of the disclosure, intervertebral disc cartilage or joint cartilage is repaired. One aspect of the disclosure is to use HDFs as cell sourcing for engineering new cartilage for the intervertebral disc, because these cells are easy to harvest and to grow. Differentiation of these cells into chondrocyte-like cells is described herein.

### I. Cells Utilized in the Invention

In certain aspects described herein, any cell may be employed so long as the cell is capable of differentiating into a chondrocyte or chondrocyte-like cell. However, in the invention, the cell is a fibroblast cell, such as a dermal fibroblast, tendon fibroblast, ligament fibroblast, or synovial fibroblast, for example. Autologous cells may be utilized, although in alternative embodiments allogeneic cells are employed; in specific embodiments, the allogeneic cells have been assayed for disease and are considered suitable for human transmission. In certain aspects of the invention, the cell or cells are autologous, although in alternative embodiments the cells are allogeneic. In cases wherein the cells are not autologous, prior to use in the invention the cells may be processed by standard means in the art to remove potentially hazardous materials, pathogens, *etc.*

The rationale for using autologous HDFs as a means of cell sourcing follows from the following: 1) HDFs can be non-invasively harvested from a punch biopsy as little as a 3.0mm diameter circular skin specimen, for example; 2) the risk of contamination from another donor (such as Hepatitis B Virus, Human Immunodeficiency Virus, Creutzfeldt-Jakob disease, *etc*.) does not exist.; and 3) HDFs can expand easily in culture and differentiate into chondrocyte-like cells under particular culture conditions. Other fibroblast populations could be used, such as tendon or ligament, for example. In an embodiment, autologous fibroblasts are preferred. Some aspects of the invention may employ HDFs purchased commercially, such as from laboratories (such as Cascade Biologics). The cells can be adult HDFs or neonatal HDFs. Neonatal foreskin fibroblasts are a very convenient source of cells, for example. These cells are used commercially and are readily available and easy to grow.

As described herein, autologous HDFs are harvested from punch biopsy of skin tissue (6 mm) from the individual. In the laboratory, subcutaneous fat and deep dermis may be dissected away with scissors. The remaining tissue may be minced and incubated overnight in 0.25% trypsin at 4°C. Then, dermal and epidermal fragments may be separated, such as mechanically separated. The dermal fragments of the biopsy may be minced and the pieces may be used to initiate explant cultures. Fibroblasts harvested from the explants may be grown in Dulbecco's MEM (DMEM) with 10% calf serum at 37°C in 8% CO₂. These cells may be expanded before being differentiated into chondrocytes, in particular aspects.

In particular aspects, chondrocyte-like differentiation of human dermal fibroblasts may be facilitated by employing mechanical strain. In specific embodiments of the invention, upon differentiation from fibroblasts, the resultant cells in vivo comprise expression of certain biochemical markers indicative of type I and II collagen and proteoglycans.

In particular aspects, chondrocyte-like differentiation of human dermal fibroblasts may occur in vivo, in which the micro-environment of the intervertebral disc is conducive for chondrocytic differentiation. Hydrostatic loading, hypoxia, cell to cell interaction with resident chondrocytic cells in the disc and other biochemical environments in the intervertebral disc may facilitate differentiation from fibroblast to chondrocytic cells, in particular embodiments. In specific embodiments of the invention, the cells in the intervertebral disc following cell transplantation will be a combination of fibrocytic and chondrocytic cells that produce both fibrous and chondrocytic tissues with biochemical markers of both type I and type II collagen and/or a number of proteoglycans found in cartilaginous and fibrous tissues.

### II. Aspects of Exemplary Methods of the Disclosure, including Methods of Repairing Damaged Cartilage

Described herein are methods of differentiating cells, including fibroblasts (for example, human) into chondrocyte-like cells in vivo. The methods may comprise the step of delivering fibroblasts to a joint of an individual, wherein prior to delivering the fibroblasts are not subjected to growth factors, matrix molecules, mechanical strain, or a combination thereof. The fibroblasts may or may not be exposed to hypoxic conditions prior to delivery in vivo.

Mechanical stress /strain are important factors for chondrogenesis. The method described herein uses *in vivo* mechanical strains and, in particular aspects, uses inherent pressure from the spine to provide mechanical strain. In some aspects, the method occurs in the absence of other types of pressure, including intermittent hydrostatic pressure, shear fluid stress, and so forth. In some aspects, the method occurs in the absence of pressure other than inherent spinal pressure, low oxygen tension, growth factors, culturing in a matrix, and so forth. In some aspects, pressure load from the spine is employed to induce differentiation of fibroblasts to other cells.

Fibroblasts can be obtained from donor source (allogenic) or autologous skin biopsy. Isolating cells from the skin and expanding them in culture may be employed, and in certain cases the cells are not manipulated or are minimally manipulated (for example, exposed to serum, antibiotics, *etc*). These cells can be put into a device (for example, a syringe having resuspended cells in media from a monolayer culture) and injected into the individual. Serum that is used to feed the cells for multiplication may be washed out with media such as DMEM to avoid any extraneous serum to be injected into the individual. In aspects of this system, there is no matrix employed, including no alginate. In aspects described herein, one injects the cells only (or a minimal amount of fluid to suspend the cells for injection) and does not inject media, for example. The fluid suspension that contains the cells may comprise buffer, amino acids, salts, glucose and/or vitamins that are components of DMEM. Exemplary matrix molecules for cell manipulation that are not employed in method steps described herein include polymers (including PGA, PLGA, and PCL, for example); natural hydrogels such as collagen, hyaluronic acid, alginate, agarose, chitosan, for example; and synthetic hydrogels such as PEO, PVA, PAA, *etc*.).

In specific aspects of the invention, cells are induced to undergo differentiation into chrondrocytes or chondrocyte-like cells. Such differentiation occurs subsequent to delivery in vivo. In specific embodiments of the invention, mechanical stress stimulates chondrogenic differentiation of HDFs.

In aspects of the invention, one can improve the matrix biomechanics and biology of the disc by increasing the disc size, collagen content, and/or level of certain biological molecules. Cells in the discs, as long as they do not leak out of the space and do not die, produce matrix molecules such as collagen, proteoglycan, *etc.,* in embodiments of the invention. In certain aspects, the biological molecules provide beneficial biomechanical properties, such as resisting compression/tension loadings. Cells subjected to loading with normal standing/walking/bending of the spine will differentiate into cartilaginous cells or cartilaginous-like cells *in vivo.* Both fibroblasts and chondrocytic cells in the disc may produce fibrous and/or cartilage matrix or tissue that can improve the intervertebral disc height and volume and enhance biomechanical properties.

In some methods described herein, following obtaining of the fibroblast cells one may expand the number of cells, although in alternative embodiments fibroblasts are provided *in vivo* to an individual in need thereof in the absence of any prior expansion. The skilled artisan recognizes that cells in culture require nutrition and one can feed the cells with media, such as FBS (fetal bovine serum). Contamination or infection may be prevented (for example, by adding antibiotics), in some cases. Prior to injection of the cells to the individual, the cells are washed with DMEM media to remove FBS and antibiotics, for example, and the cells in suspension will be used for injection. The fluid suspension may contain a small amount of media including buffer, amino acids, salts, glucose and/or vitamins, for example. *In vitro* growth of the fibroblast cells may comprise at least one or more days for growth prior to use *in vivo.* In certain cases, the cells may be checked or monitored to ensure that at least some of the cells are dividing. Cells that are not dividing may be removed.

In certain aspects, disc height is improved and/or certain biochemical markers are exhibited in the implanted cells. The disc height can be measured using plain radiographs, comparing before and after therapy, for example. In at least specific cases, one can also employ magnetic resonance imaging (MRI), biochemical marker assay, and/or histology. Restoring disc height improves the space for the spinal nerves that are crossing the spine, and it has an indirect benefit in this way in addition to improving the disc biomechanics and biology of the area. Histological changes following transplantation of the fibroblasts can show a combination of fibrous and cartilaginous cells and matrix with increased disc height because of more abundant tissue, in particular embodiments.

In some aspects, fibroblasts cells are injected between the vertebrae or intervertebral discs, and the cells in the nucleus pulposus may migrate to the fissures in the annulus associated disc degeneration. These cells will enhance matrix formation in both nucleus pulposus and anulus fibrosus to aid in repair and tissue regeneration. The cells in the nucleus pulposus will differentiate more toward chondrocytic and the cells in the annulus fibrosus will be more fibrocytic due to mechanical and biochemical environments of the nucleus pulposus and annulus fibrosus.

In some aspects, differentiation of the fibroblast cells does not begin until implantation *in vivo* and not all of the transplanted cells can differentiate into chondrocytic cells because of varying biomechanical and biochemical environments.

In aspects described herein for illustrative purposes, one obtains fibroblasts, for example from the individual being treated, obtains them from another individual (including a cadaver or living donor, for example), or obtains them commercially. One can take a skin biopsy and in some embodiments may manipulate the skin biopsy. For example, one can digest the skin tissue overnight to get fibroblasts, culture the cells to expand, and provide them to the individual, including by injecting them into the individual. Prior to delivery to the individual, the cells may be passaged one or more times depending on the number of cells needed, including 1, 2, 3, 4, 5, 6, 7, 8, 9, 10, or more times, for example. Passaging may occur over the course of one or more days, including 2, 3, 4, 5, 6, 7, 8, 9, or 10 days, or 1, 2, 3, 4, or more weeks, for example. In some embodiments, the cells are passaged for 5-7 days, for example.

In aspects of thedisclosure, intervertebral disc disease is prevented by providing fibroblasts in vivo to an individual in need thereof, including an individual susceptible to the disease, for example an aging individual. In some embodiments, the individual is an adult. An individual at risk for the disease includes an athlete (professional or recreational), smokers, obese individuals, and/or those whose occupations or lifestyle require physical labor, including excessive lifting, for example.

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### EXAMPLE 1

### FIBROBLAST INJECTION AND IN VIVO DIFFERENTIATION

In aspects described herein, fibroblasts are delivered to mammalian vertebrae to improve intervertebral disc degeneration, for example. In some aspects, fibroblasts are delivered to mammalian vertebrae to induce chondrocyte differentiation or to continue chondrocyte differentiation.

A rabbit model was employed that involves puncturing the annulus, which reduces the disc height (due to matrix loss and degeneration, for example) to about 70% normal height about 4 weeks after the injury. The cell transplantation in this model is performed at 4 weeks following the annulus puncture, and the disc height gradually increases, for example for the next 3-4 weeks. The cells that were injected are contained in the disc and are alive to make more matrix (fibrous and cartilaginous tissue) to increase the disc height. The more matrix and increased disc height results in better biomechanical function and less pain for the individual. In specific embodiments, for example based on MRI, regenerated tissue is mostly fibrocartilage rather than hyaline type cartilage with high proteoglycans and water. In certain aspects, biochemical analysis shows that type I and type II collagen is expressed, which shows that there is cartilaginous component, indicating that at least in some cases there is cartilaginous tissue (if it were all fibrous (scar tissue), type I collagen without type II collagen would be mainly expressed, but cartilaginous tissue expresses type II collagen).

Upon manipulation of the above-referenced rabbit model, the disc height increases following transplantation of the fibroblasts.

## Claims

1. Fibroblasts for use in a method of treating an individual in need of cartilage repair, the method comprising differentiating human fibroblasts into chondrocyte-like cells *in vivo* by delivering fibroblasts to a joint of an individual, wherein prior to delivery, the fibroblasts are not subjected to growth factors, matrix molecules, mechanical strain, or a combination thereof.

2. The fibroblasts for use of claim 1, wherein the fibroblasts are tendon fibroblasts, ligament fibroblasts, synovial fibroblasts, foreskin fibroblasts or a mixture thereof.

3. The fibroblasts for use of claim 1 or 2, wherein:
(a) the individual has intervertebral disc disease; and/or
(b) the joint is an intervertebral disc.

4. The fibroblasts for use of any one of the preceding claims, wherein the fibroblasts are delivered between intervertebral discs, optionally wherein the fibroblasts are delivered between or in nucleus pulposus and fissures in the inner annulus fibrosus.

5. The fibroblasts for use of any one of the preceding claims, wherein the fibroblasts are obtained from the individual.

6. The fibroblasts for use of claim 5, wherein:
(a) the obtained fibroblasts are expanded, optionally for at least one day; and/or
(b) the obtained fibroblasts are passaged, optionally more than once.

7. The fibroblasts for use of any one of the preceding claims, wherein following delivery of the fibroblasts to the joint of the individual, a plurality of fibroblasts die.

8. The fibroblasts for use of claim 7, wherein death of the fibroblasts results in a cellular response from endogenous joint cells of the individual, optionally wherein the cellular response comprises stimulation of growth of the endogenous joint cells of the individual.

9. The fibroblasts for use of any one of the preceding claims, wherein following delivery of the fibroblasts to the joint, the fibroblasts triggers a cellular response from endogenous joint cells of the individual, optionally wherein the cellular response comprises stimulation of growth of the endogenous joint cells of the individual.

10. The fibroblasts for use of any one of the preceding claims, wherein the delivery of the fibroblasts to the joint of the individual indirectly or directly results in the development of scar tissue in the joint.

## Patentansprüche

1. Fibroblasten für die Verwendung in einem Verfahren zum Behandeln eines Individuums, das eine Knorpelreparatur benötigt, wobei das Verfahren das Differenzieren menschlicher Fibroblasten in chondrozytenähnliche Zellen *in vivo* durch Abgeben von Fibroblasten in ein Gelenk eines Individuums umfasst, wobei die Fibroblasten vor der Abgabe Wachstumsfaktoren, Matrixmolekülen, mechanischer Beanspruchung oder einer Kombination davon nicht ausgesetzt werden.

2. Fibroblasten für die Verwendung nach Anspruch 1, wobei die Fibroblasten Sehnenfibroblasten, Bänderfibroblasten, Synovialfibroblasten, Vorhautfibroblasten oder ein Gemisch davon sind.

3. Fibroblasten für die Verwendung nach Anspruch 1 oder 2, wobei:
(a) das Individuum an einer Bandscheibenkrankheit leidet; und/oder
(b) das Gelenk eine Bandscheibe ist.

4. Fibroblasten für die Verwendung nach einem der vorhergehenden Ansprüche, wobei die Fibroblasten zwischen die Bandscheiben abgegeben werden, optional wobei die Fibroblasten zwischen die Bandscheiben oder in den Nucleus pulposus und die Fissuren in dem inneren Annulus fibrosus abgegeben werden.

5. Fibroblasten für die Verwendung nach einem der vorhergehenden Ansprüche, wobei die Fibroblasten von dem Individuum erhalten werden.

6. Fibroblasten für die Verwendung nach Anspruch 5, wobei:
(a) die erhaltenen Fibroblasten expandiert werden, optional für wenigstens einen Tag; und/oder
(b) die erhaltenen Fibroblasten, optional mehr als einmal, durchgelassen werden.

7. Fibroblasten für die Verwendung nach einem der vorhergehenden Ansprüche, wobei nach Abgabe der Fibroblasten an das Gelenk des Individuums eine Mehrzahl von Fibroblasten abstirbt.

8. Fibroblasten für die Verwendung nach Anspruch 7, wobei das Absterben der Fibroblasten zu einer zellulären Reaktion von endogenen Gelenkzellen des Individuums führt, optional wobei die zelluläre Reaktion eine Stimulation des Wachstums der endogenen Gelenkzellen des Individuums umfasst.

9. Fibroblasten für die Verwendung nach einem der vorhergehenden Ansprüche, wobei nach der Abgabe der Fibroblasten an das Gelenk, die Fibroblasten eine zelluläre Reaktion von den endogenen Gelenkzellen des Individuums auslösen, optional wobei die zelluläre Reaktion eine Stimulation des Wachstums der endogenen Gelenkzellen des Individuums umfasst.

10. Fibroblasten für die Verwendung nach einem der vorhergehenden Ansprüche, wobei die Abgabe der Fibroblasten an das Gelenk des Individuums direkt oder indirekt zu der Entwicklung von Nabengewebe in dem Gelenk führt.

## Revendications

1. Fibroblastes pour utilisation dans une méthode de traitement d'un individu nécessitant une réparation du cartilage, la méthode comprenant la différenciation *in vivo* de fibroblastes humains en cellules de type chondrocytes par administration de fibroblastes dans une articulation d'un individu, lesdits fibroblastes, avant l'administration, n'étant pas soumis à des facteurs de croissance, des molécules matricielles, des contraintes mécaniques, ou une combinaison de ceux-ci.

2. Fibroblastes pour utilisation selon la revendication 1, lesdits fibroblastes étant des fibroblastes de tendon, des fibroblastes de ligament, des fibroblastes synoviaux, des fibroblastes de prépuce ou un mélange de ceux-ci.

3. Fibroblastes pour utilisation selon la revendication 1 ou 2, dans le cadre de laquelle utilisation :
(a) l'individu a une discopathie, et/ou
(b) l'articulation est un disque intervertébral.

4. Fibroblastes pour utilisation selon l'une quelconque des revendications précédentes, lesdits fibroblastes étant administrés entre des disques intervertébraux, lesdits fibroblastes étant facultativement administrés entre ou dans des nucleus pulposus et des fissures de l'annulus fibrosus interne.

5. Fibroblastes pour utilisation selon l'une quelconque des revendications précédentes, lesdits fibroblastes étant obtenus auprès de l'individu.

6. Fibroblastes pour utilisation selon la revendication 5, dans le cadre de laquelle utilisation :
(a) les fibroblastes obtenus sont développés, facultativement pendant au moins une journée, et/ou
(b) les fibroblastes obtenus sont repiqués, facultativement plus d'une fois.

7. Fibroblastes pour utilisation selon l'une quelconque des revendications précédentes, dans le cadre de laquelle utilisation, après l'administration desdits fibroblastes dans l'articulation de l'individu, une pluralité de fibroblastes meurt.

8. Fibroblastes pour utilisation selon la revendication 7, la mort des fibroblastes provoquant une réponse cellulaire chez les cellules d'articulation endogènes de l'individu, ladite réponse cellulaire comprenant facultativement la stimulation de la croissance des cellules d'articulation endogènes de l'individu.

9. Fibroblastes pour utilisation selon l'une quelconque des revendications précédentes, dans le cadre de laquelle utilisation, après l'administration desdits fibroblastes dans l'articulation, les fibroblastes déclenchent une réponse cellulaire chez les cellules d'articulation endogènes de l'individu, ladite réponse cellulaire comprenant facultativement la stimulation de la croissance des cellules d'articulation endogènes de l'individu.

10. Fibroblastes pour utilisation selon l'une quelconque des revendications précédentes, l'administration desdits fibroblastes dans l'articulation de l'individu provoquant indirectement ou directement le développement de tissus cicatriciels dans l'articulation.
